# EUROPÄISCHE PATENTSCHRIFT

(11) **EP 1 507 589 B1**
(45) Veröffentlichungstag und Bekanntmachung des Hinweises auf die Patenterteilung: **21.06.2006**
(21) Anmeldenummer: 03725132.9
(22) Anmeldetag: 02.05.2003
(51) Int. Cl.: B01L 3/00, G01N 33/543

(54) **VORRICHTUNG ZUR PROBENNAHME VON FLÜSSIGEN PROBEN**
SAMPLING DEVICE FOR LIQUID SAMPLES
DISPOSITIF DE PRELEVEMENT D'ECHANTILLONS LIQUIDES

(30) Priorität: 07.05.2002 DE 10220296
(43) Veröffentlichungstag der Anmeldung: 23.02.2005
(73) Patentinhaber: Roche Diagnostics GmbH, 68305 Mannheim (DE); F. HOFFMANN-LA ROCHE AG, 4070 Basel (CH)
(72) Erfinder: GERSTLE, Volker, 64653 Lorsch (DE); UNKRIG, Volker, 68526 Ladenburg (DE); AUGSTEIN, Manfred, 68259 Mannheim (DE)
(86) Internationale Anmeldenummer: PCT/EP2003/004600
(87) Internationale Veröffentlichungsnummer: WO 2003/095092

(56) Entgegenhaltungen:
- DE-A- 19 753 847
- DE-A- 19 753 850
- US-A- 4 810 658
- US-A- 5 192 502
- US-B1- 6 270 637

## Beschreibung

Die Erfindung betrifft eine Vorrichtung zur Probennahme von flüssigen Proben, bei der die Probe in einem kapillaraktiven Kanal von einem Probennahmeort zu einem Bestimmungsort transportiert wird.

Zur schnellen und einfachen qualitativen oder quantitativen analytischen Bestimmung von Bestandteilen von flüssigen Proben, z. B. wässrigen Körperflüssigkeiten, wie Blut, Serum oder Urin, werden oft sogenannte trägergebundene Tests (Testträger, Testelemente, Teststreifen) verwendet. Bei diesen trägergebundenen Tests sind Nachweisreagenzien in entsprechenden Schichten eines Trägers eingebettet, der mit der flüssigen Probe in Kontakt gebracht wird. Die Reaktion von flüssiger Probe und Reagenzien führt bei Anwesenheit eines Zielanalyten zu einem nachweisbaren Signal, z. B. einem messbaren elektrischen Signal oder einem Farbumschlag, welcher visuell oder mit Hilfe eines Geräts, z. B. reflektionsphotometrisch, ausgewertet werden kann.

Trägergebundene Tests sind häufig als Teststreifen ausgebildet, die im wesentlichen aus einer länglichen Tragschicht aus Kunststoffmaterial und darauf angebrachten Nachweisschichten aus Testfeldern bestehen. Es sind jedoch auch Testträger bekannt, die als quadratische oder rechteckige Plättchen gestalte sind.

In neuerer Zeit werden insbesondere Teststreifen angeboten, die einen kapillaraktiven Spalt (Kapillarspalt) enthalten, welcher Probenflüssigkeit von einem Ende des Teststreifens (Probennahmeort oder Probenaufgabeort) zu den Reagenzienzonen befördert, welche typischerweise in einem Abstand von einigen wenigen Zentimetern vom Probennahmeort untergebracht sind. Dadurch wird beispielsweise ermöglicht, die Probe, insbesondere eine Blutprobe, auf einen Teststreifen aufzugeben, der sich in einem Auswertegerät befindet, ohne das Auswertegerät der Gefahr der Kontamination durch die Blutprobe auszusetzen. Typische Vertreter solcher Teststreifen sind beispielsweise in den folgenden Patentdokumenten beschrieben:

EP-A 0 359 831; US 6,071,391; US 6,156,173; WO-A 00/20626; WO-A 99/29428; WO-A 99/29429; EP-A 0 170 375.

Die in den oben genannten Dokumenten beschriebenen Testelemente bestehen im wesentlichen aus einem Träger, einer Abdeckung und einer zwischen Träger und Abdeckung liegenden Zwischenschicht, welche zusammen den kapillaraktiven Kanal bilden. In einem abgegrenzten Bezirk innerhalb des kapillaraktiven Kanals befinden sich Reagenzien, welche für den Nachweis des Zielanalyten oder Zielparameters der Blut- oder Flüssigkeitsprobe erforderlich sind. Die trägergebundenen Tests des Standes der Technik besitzen alle einen klar definierten und eng begrenzten Bereich, in dem Probenmaterial aufgegeben werden kann, sodass eine Befüllung des Kapillarkanals erfolgt. Dieser Bereich befindet sich entweder am Ende oder an einer oder beiden Seitenkanten des Testträgers. Es sind auch Testträger bekannt, bei denen eine Dosierung des flüssigen Probenmaterials durch eine Öffnung im Träger oder in der Abdeckung quasi von oben oder unten erfolgt. Im englischsprachigen Sprachraum werden die Varianten des Probenbefüllens an unterschiedlichen Stellen mit "front dosing", "side dosing" und "top dosing" bezeichnet.

Bei Teststreifen, die von ungeschultem Personal zu bedienen sind, beispielsweise von Diabetikern oder Antikoagulationspatienten im Bereich des sogenannten Home-Monitoring haben sich Front- und Side-Dosing-Varianten der Probenbefüllung wegen der einfachen Handhabbarkeit (meist wird ein Blutstropfen aus der Fingerkuppe an den Teststreifen herangeführt) als bevorzugt herausgestellt. Im professionellen Bereich (Arztpraxen, medizinischen Labors etc.) hingegen werden Teststreifen mit Top-Dosing-Variante bevorzugt, da hier ein Blutauftrag meistens mit Applikationshilfsmitteln, wie z. B. Pipetten oder Kapillaren, vorgenommen wird und mit diesen Hilfsmitteln ein front oder side dosing nur sehr schwer zu bewerkstelligen ist.

Es mangelt bislang an trägergebundenen Tests, die sowohl im Home-Monitoring-Bereich als auch im professionellen Bereich gleichermaßen vorteilhaft anzuwenden sind.

Aufgabe der vorliegenden Erfindung war es, die Nachteile des Standes der Technik zu beseitigen. Insbesondere war es die Aufgabe, eine Vorrichtung zur Probennahme von flüssigen Proben bereitzustellen, bei der sowohl eine bequeme Probenapplikation mit Applikationshilfsmitteln, wie Pipetten oder Kapillaren, möglich ist, als auch eine Dosierung von Probenflüssigkeit (insbesondere von Blut) von Körperoberflächen.

Diese Aufgabe wird durch den Gegenstand der Erfindung, wie er in den Patentansprüchen charakterisiert ist, gelöst. Gegenstand der Erfindung ist eine Vorrichtung zur Probennahme von flüssigen Proben, bei der die Probe in einem kapillaraktiven Kanal von einem Probennahmeort zu einem Bestimmungsort transportiert wird und bei der der kapillaraktive Kanal im wesentlichen von einem Träger, einer Abdeckung und einer zwischen Träger und Abdeckung liegenden Zwischenschicht gebildet wird, wobei der Träger im Bereich des Probennahmeortes über die Abdeckung hinausragt. Wesentlich ist dabei, dass die Zwischenschicht, welche die Geometrie des kapillaraktiven Kanals bestimmt, im Bereich des Probennahmeortes in Richtung des Bestimmungsortes nach hinten versetzt ist, sodass sowohl Träger als auch Abdeckung über die Zwischenschicht hinausragen. Dadurch wird im Bereich des Probennahmeortes eine Öffnung geschaffen, die im wesentlichen die gesamte Breite der Vorrichtung einnimmt. Die Höhe der Zwischenschicht bestimmt dabei die Kapillaraktivität des Kapillarkanals. Sie ist so zu wählen, dass Kapillarität entsteht. Typischerweise ist die Dicke der Zwischenschicht einige wenige Hundert µm. In bevorzugten Ausführungsformen der Erfindung können entweder Träger und Zwischenschicht oder Abdeckung und Zwischen oder Träger, Abdeckung und Zwischenschicht aus einem Stück gefertigt sein.

Typischerweise sind Träger und Abdeckung Folien aus einem Kunststoffmaterial, während die Zwischenschicht ein beidseitiges Klebeband geeigneter Dicke ist.

Typische Vertreter der erfindungsgemäßen Vorrichtung sind insbesondere analytische Testelemente (Teststreifen, Biosensoren), Küvetten oder Probennahmeelemente, wie Pipetten oder ähnliches.

Vorzugsweise ist die erfindungsgemäße Vorrichtung ein analytisches Testelement, bei dem bereits während oder nach der Aufnahme der Probenflüssigkeit geeignete Nachweisreaktionen ablaufen, welche die Bestimmung der Anwesenheit oder Menge eines Analyten in der Probe erlauben oder dazu geeignet sind, bestimmte Probeneigenschaften zu detektieren. Analytische Testelemente im hier benutzten Sinn sind visuell oder apparativ-optisch auswertbare Testelemente, z. B. Teststreifen, Biosensoren, wie z. B. enzymatische Biosensoren oder optische Biosensoren (Optroden, Wellenleiter etc.), elektrochemische Sensoren und dergleichen mehr. Vorzugsweise werden in den analytischen Testelementen enzymatische, immunologische oder auf Nukleinsäuren basierende Methoden zum Analytnachweis eingesetzt. Die Vorrichtung zur Probennahme im erfindungsgemäßen Sinn kann jedoch auch eine Küvette oder Pipette sein, die lediglich der Probenaufnahme dient und bei der die Probe zur Analyse entweder wieder abgegeben wird oder bei der die Analyse ohne nachgeschaltete Reaktionen erfolgt. Die Vorrichtung zur Probennahme im erfindungsgemäßen Sinn kann selbstverständlich auch zur Lagerung und Aufbewahrung von Probenflüssigkeit benutzt werden. Derartige Vorrichtungen zur Probennahme sind in ihren Grundzügen im Stand der Technik umfassend beschrieben und dem Fachmann in einer Vielzahl von Ausführungsformen geläufig.

Der kapillaraktive Kanal oder Kapillarkanal der erfindungsgemäßen Vorrichtung dient dazu, die flüssige Probe von einem ersten Ort der Vorrichtung zu einem davon entfernt gelegenen zweiten Ort zu transportieren. Der erste Ort soll im hier benutzten Sinn der Probennahmeort sein; der zweite wird als Bestimmungsort bezeichnet.

Bei streifenförmigen Testelementen entspricht der Probennahmeort beispielsweise im wesentlichen einer der kurzen Kanten oder Randflächen des Testelements. Der Bestimmungsort entspricht beispielsweise im wesentlichen dem Ort, an dem die Nachweisreaktion für den Zielanalyten beobachtet wird und welcher in aller Regel die Nachweisreagenzien trägt. Allgemein ausgedrückt ist der Bestimmungsort meist das dem Probennahmeort entgegengesetzte Ende des kapillaraktiven Kanals.

In einer bevorzugten Ausführungsform der erfindungsgemäßen Vorrichtung sind eine oder mehrere oder alle dem Kapillarkanal zugewandten Oberflächen des Trägers, der Abdeckung und der Zwischenschicht hydrophil ausgestaltet.

Durch die erfindungsgemäße Eigenschaft, wonach der Träger im Bereich des Probennahmeortes über die Abdeckung hinausragt, stellt der Träger eine flächige Applikationszone bereit, die ein leichtes Aufbringen der Probe mittels Applikationshilfsmitteln, wie z. B. Pipetten oder Kapillaren ermöglicht.

Die erfindungsgemäße Eigenschaft, wonach die Zwischenschicht der Vorrichtung im Bereich des Probennahmeortes in Richtung des Bestimmungsortes nach hinten versetzt ist, sodass sowohl Träger als auch Abdeckung über die Zwischenschicht hinausragen, sorgt dafür, dass an den Rändern der Vorrichtung Bereiche verbleiben, die ein side dosing von Probenflüssigkeit ermöglicht.

In einer bevorzugten Ausführungsform der erfindungsgemäßen Vorrichtung ist der kapillaraktive Kanal im Bereich des Probennahmeortes aufgeweitet, vorzugsweise bis hin an zumindest eine seitliche Kante der Vorrichtung. Ganz besonders bevorzugt ist die Aufweitung trichterförmig ausgebildet. Der Trichter kann dabei im wesentlichen gerade (dreieckig) oder aber gekrümmt (trompetenförmig) gestaltet sein. Da die Geometrie des kapillaraktiven Kanals im wesentlichen durch die Zwischenschicht bestimmt wird, enthält die Zwischenschicht eine entsprechende geometrisch gestaltete Aussparung.

In der bevorzugten Ausführungsform stellt die Bodenfolie folglich eine flächige Applikationszone bereit. Diese wird durch den trichterförmig ausgeprägten Kapillaranfang begrenzt. Dieser Trichter reicht beidseitig bis an den Streifenrand. Dieser Trichter wird von der Abdeckung so überdeckt, dass sich zwischen Abdeckung, Rand der Zwischenschicht und dem Träger ein Kapillarspalt bildet.

In einer weiteren besonders bevorzugten Ausführungsform der Erfindung enthält die Vorrichtung zur Probennahme auf dem über die Abdeckung hinausragenden Teil des Trägers im Bereich des Probennahmeortes (Applikationszone) ein Mittel zur Aufnahme überschüssiger Probe. Dieses Mittel steht nicht in direktem Kontakt mit der Abdeckung. Vorzugsweise enthält dieses Mittel einen kapillaraktiven Spalt oder ein saugfähiges Material (z. B. ein Vlies, Gewebe, Gewirke, Schwamm etc.), sodass überschüssiges Probenmaterial darin aufgenommen werden kann. Vorzugsweise ist die Kapillarität dieser auch als "Waste-Zone" zu bezeichnenden Region niedriger als die Kapillarität des kapillaraktiven Kanals, welcher vom Probennahmeort zum Bestimmungsort der Probe führt, sodass Probenmaterial, welches auf die Vorrichtung aufgegeben wird, bevorzugt zunächst den kapillaraktiven Kanal füllt, welcher vom Probennahmeort zum Probenbestimmungsort führt und erst nach dessen Füllung das Mittel zur Aufnahme überschüssiger Probe befüllt wird. Vorteilhafterweise kann das Mittel zur Aufnahme überschüssiger Probe gleichzeitig als Handhabungshilfe für die erfindungsgemäße Vorrichtung dienen.

Die erfindungsgemäße Vorrichtung bietet eine Reihe von Vorteilen:
1. Die Probenapplikationsstelle ist innerhalb einer relativ großen Fläche und über die gesamte Breite des Teststreifens frei wählbar.
2. Die erfindungsgemäße Vorrichtung ist in allen Gebrauchslagen selbstdosierend.
3. Die erfindungsgemäße Vorrichtung ist sowohl von oben als auch von den Seiten mit Probe zu befüllen, was eine Applikation mit Pipetten, Kapillaren oder aber eine Probenaufgabe direkt von der Körperoberfläche (Fingerbeere, Unterarm o. ä.) ermöglicht. Insbesondere für den Fall, dass die erfindungsgemäße Vorrichtung ein analytisches Testelement ist, kann hiermit eine Bedienung unterschiedlicher Marktsegmente (Home-Monitoring, professioneller Markt) erfolgen.
4. Bezüglich der zu applizierenden Probenmenge besitzt die erfindungsgemäße Vorrichtung eine sehr große Flexibilität, da durch die Gestaltung der Applikationsfläche unterschiedliche Probenvolumina auf die Vorrichtung aufgegeben werden können, ohne das minimal benötigte Probenvolumen zu vergrößern.
5. In einer bevorzugten Ausführungsform können Mittel vorgesehen sein, die ein Überdosieren der Probenmenge verhindern, in dem überschüssige Probe sicher im Inneren der Vorrichtung aufgenommen werden kann.

Die Erfindung wird durch die beiliegenden Figuren näher erläutert:
Figur 1 zeigt eine schematische Aufsicht auf ein erfindungsgemäßes Testelement.
Figur 2 zeigt schematisch die einzelnen, beim Aufbau des Testelements aus Figur 1 beteiligten Schichten.
Figur 3 zeigt einen vergrößerten Ausschnitt aus dem Testelement aus Figur 1 im Bereich des Probennahmeortes in einer Seitenansicht.

Die Ziffern in den Figuren bedeuten:
- 1:: Vorrichtung zur Probennahme (hier: Testelement)
- 2, 2':: kapillaraktiver Kanal
- 3:: Probennahmeort
- 4:: Bestimmungsort
- 5:: Träger
- 6, 6':: Abdeckung
- 7, 7':: Zwischenschicht
- 8:: Mittel zur Aufnahme überschüssiger Probe (Waste-Zone)
- 9:: Entlüftungsöffnung
- 10:: Elektrodenstrukturen

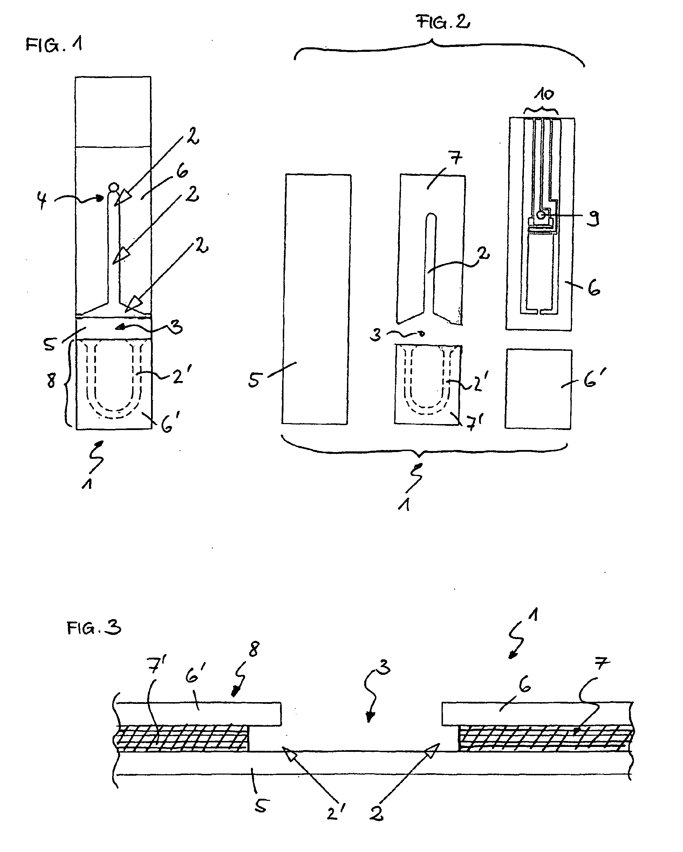

In Figur 1 ist schematisch eine Aufsicht auf das erfindungsgemäße analytische Testelement (1) abgebildet. Wie aus Figur 1 im Zusammenspiel mit Figur 2 zu entnehmen ist, ist das analytische Testelement (1) aus einem Träger (5) aufgebaut, auf dem eine Zwischenschicht (7) in Form eines doppelseitigen Klebebands aufgeklebt ist. Die Zwischenschicht (7) enthält eine Aussparung für den kapillaraktiven Kanal (2), welcher in der hier gezeigten Ausführungsform im Bereich des Probennahmeortes (3) trichterförmig aufgeweitet ist. Außerdem ist auf dem Träger (5) eine zweite Zwischenschicht (7') aufgebracht, welche optional einen zweiten kapillaraktiven Kanal (2') (gestrichelt) enthalten kann. Zwischenschicht (7') ist in der in den Figuren gezeigten Ausführungsform ebenfalls ein doppelseitiges Klebeband, auf welches eine Abdeckung (6') zur Vereinfachung der Handhabung des Testelements (1) aufgeklebt ist.

Auf die Zwischenschicht (7) ist die Abdeckung (6) aufgeklebt, welche in der hier gezeigten Ausführungsform eine Entlüftungsöffnung (9) sowie Elektrodenstrukturen (10) enthält. Die Entlüftungsöffnung (9) ermöglicht das Entweichen von Luft bei der Befüllung des Kapillarkanals (2). Die Elektrodenstrukturen (10) enthalten im Bereich des Bestimmungsorts (4) für die Probenflüssigkeit Strukturen für Arbeits- und Gegenelektroden. Der in Figur 1 gezeigte Testträger (1) kann beispielsweise für amperometrische Analytbestimmungen eingesetzt werden, beispielsweise um bestimmte Blutparameter (Glucose, Lactat, Cholesterin etc.) oder Bluteigenschaften (Hämatokrit, Gerinnungszeiten) zu bestimmen.

Selbstverständlich ist es auch möglich, dass alternativ zu den Elektrodenstrukturen (10) Reagenzien für einen optischen, insbesondere reflektionsphotometrischen Nachweis von Analyten im Bereich des Bestimmungsorts (4) untergebracht sind. Zu diesem Zweck ist es vorteilhaft, dass entweder der Träger (5) oder die Abdeckung (6) zumindest im Bereich des Bestimmungsorts (4) transparent sind.

Wie insbesondere aus Figur 3 ersichtlich ist, ist die Zwischenschicht (7) (und in der gezeigten Ausführungsform auch die Zwischenschicht 7') im Bereich des Probennahmeortes (3), d. h. der Stelle, an der Probenflüssigkeit auf das Testelement (1) aufgegeben wird, nach hinten eingerückt (d. h. weg vom Probennahmeort (3) eingerückt). Träger (5) und Abdeckung (6) (im hier gezeigten Fall auch Abdeckung 6') ragen im Bereich des Probennahmeortes (3) über die Zwischenschicht (7) (im hier gezeigten Fall auch über die Zwischenschicht 7') hinaus. Dadurch wird auch eine seitliche Dosierung von Probenflüssigkeit ermöglicht. Zwischen Träger (5) und Abdeckung 6, 6', bildet sich ein Kapillarspalt, der sich bis an den Rand des Testelements (1) erstreckt. Eine Befüllung des Kapillarkanals (2) ist somit sowohl von der Seite (side dosing) als auch von oben durch Ablegen eines Aliquots einer Blutprobe auf die freiliegende Fläche des Trägers (5) im Bereich der Probenaufgabezone (3) möglich.

Eventuelle überschüssige Probe wird durch den Kapillarkanal (2'), welche Teil des Mittels zur Aufnahme überschüssiger Probe (8) ist, vom Probennahmeort (3) abgeführt. Das Mittel zur Aufnahme überschüssiger Probe (8) verschließt gleichsam überschüssige Probe und verhindert eine Kontamination der Umgebung. Gleichzeitig kann die Zone, in der das Mittel (8) liegt, als Handhabungshilfe für das Testelement (1) dienen.

Vorzugsweise ist die Kapillarität des Mittels (8) geringer als die Kapillarität des Kapillarkanals (2), sodass Probenflüssigkeit, welche im Bereich (3) auf das Testelement (1) aufgegeben wird, zunächst überwiegend bevorzugt in den Kapillarkanal eindringt, und nur Probe, die nicht in den Kapillarkanal (2) einzudringen vermag, weil dieser bereits gefüllt ist, in das Mittel (8) aufgenommen wird.

Zur Steuerung der Kapillarität der miteinander konkurrierenden Bereiche Kapillarkanal (2) und Waste-Zone (8) kann beispielsweise die Verwendung von unterschiedlich hydrophilen Materialien zum Aufbau der Kapillare oder eine Variation der Kapillarspalthöhe dienen.

Weitere bevorzugte Ausführungsformen, die in den Figuren nicht gezeigt sind, können Elemente enthalten, die eine leichtere Identifizierbarkeit der Probenaufgabestellen für den Benutzer bewirken. Beispielsweise kann eine oder beide seitlichen Kanten des streifenförmigen Testelements aus Figur 1 im Bereich der Probenaufgabezone halbrunde oder kerbenförmige Ausnehmungen aufweisen, die eine Mulde für die Platzierung der Fingerspitze bilden und so neben einer visuellen Sichtbarmachung der Probenaufgabenstelle auch eine taktile Identifizierung dieser Stelle ermöglichen. Ebenfalls ist es möglich, dass die Abdeckung im Bereich der Probenaufgabestelle markiert ist, z. B. durch eine entsprechend platzierte Kerbe.

## Patentansprüche

1. Vorrichtung (1) zur Probennahme von flüssigen Proben, bei der die Probe in einem kapillaraktiven Kanal (2) von einem Probennahmeort (3) zu einem Bestimmungsort (4) transportiert wird und bei der der kapillaraktive Kanal (2) im wesentlichen von einem Träger (5), einer Abdeckung (6) und einer zwischen Träger (5) und Abdeckung (6) liegenden Zwischenschicht (7) gebildet wird, die die Geometrie des kapillaraktiven Kanals bestimmt, wobei der Träger (5) im Bereich des Probennahmeortes (3) über die Abdeckung (6) hinausragt, **dadurch gekennzeichnet, dass**
die Zwischenschicht (7) im Bereich des Probennahmeortes (3) in Richtung des Bestimmungsortes (4) nach hinten versetzt ist, so dass sowohl Träger (5) als auch Abdeckung (6) über die Zwischenschicht (7) hinausragen.

2. Vorrichtung gemäß Anspruch 1, **dadurch gekennzeichnet, dass**
Träger und Zwischenschicht oder Abdeckung und Zwischenschicht oder Träger und Abdeckung und Zwischenschicht aus einem Stück gefertigt sind.

3. Vorrichtung gemäß Anspruch 1 oder 2, **dadurch gekennzeichnet, dass**
der kapillaraktive Kanal im Bereich des Probennahmeortes aufgeweitet ist.

4. Vorrichtung gemäß Anspruch 3, **dadurch gekennzeichnet, dass**
der kapillaraktive Kanal im Bereich des Probennahmeortes bis an zumindest eine seitliche Kante der Vorrichtung aufgeweitet ist.

5. Vorrichtung gemäß Anspruch 4, **dadurch gekennzeichnet, dass**
der kapillaraktive Kanal im Bereich des Probennahmeortes bis an beide seitlichen Kanten der Vorrichtung aufgeweitet ist.

6. Vorrichtung gemäß 3, 4 oder 5, **dadurch gekennzeichnet, dass**
der kapillaraktive Kanal im wesentlichen trichterförmig aufgeweitet ist.

7. Vorrichtung gemäß einem der vorigen Ansprüche, **dadurch gekennzeichnet, dass**
auf dem über die Abdeckung hinausragenden Teil des Trägers im Bereich des Probennahmeortes ein Mittel (8) zur Aufnahme überschüssiger Probe angebracht ist, welche nicht in direktem Kontakt mit der Abdeckung steht.

8. Vorrichtung gemäß Anspruch 7, **dadurch gekennzeichnet, dass**
das Mittel (8) zur Aufnahme überschüssiger Probe eine geringere Kapillarität als der kapillaraktive Kanal (2) besitzt.

9. Vorrichtung gemäß Anspruch 7 oder 8, **dadurch gekennzeichnet, dass**
das Mittel (8) einen kapillaraktiven Spalt oder ein saugfähiges Material enthält.

## Claims

1. Device (1) for sampling liquid samples in which the sample is transported in a capillary-active channel (2) from a sampling site (3) to a determination site (4) and in which the capillary-active channel (2) is essentially formed by a carrier (5), a cover (6) and an intermediate layer (7) which intermediate layer (7) determines the geometry of the capillary-active channel (2) and is located between the carrier (5) and cover (6) where the carrier (5) protrudes beyond the cover (6) in the area of the sampling site (3),
**characterized in that**
the intermediate layer (7) is displaced towards the back in the direction of the determination site (4) in the area of the sampling site (3) so that the carrier (5) as well as the cover (6) protrude beyond the intermediate layer (7).

2. Device as claimed in claim 1, **characterized in that**
the carrier and intermediate layer or cover and intermediate layer or carrier and cover and intermediate layer are manufactured from one piece.

3. Device as claimed in claim 1 or 2, **characterized in that**
the capillary-active channel is widened in the area of the sampling site.

4. Device as claimed in claim 3, **characterized in that**
the capillary-active channel is widened in the area of the sampling site to at least one side edge of the device.

5. Device as claimed in claim 4, **characterized in that**
the capillary-active channel is widened in the area of the sampling site to both side edges of the device.

6. Device as claimed in claims 3, 4 or 5, **characterized in that**
the capillary-active channel is essentially widened into a funnel shape.

7. Device as claimed in one of the previous claims, **characterized in that**
means (8) for taking up excess sample which is not in direct contact with the cover is mounted on the part of the carrier that protrudes beyond the cover in the area of the sampling site.

8. Device as claimed in claim 7, **characterized in that**
the means (8) for taking up excess sample has a lower capillarity than the capillary-active channel (2).

9. Device as claimed in claim 7 or 8, **characterized in that**
the means (8) contains a capillary-active gap or an absorbent material.

## Revendications

1. Dispositif (1) pour l'échantillonnage d'échantillons liquides, dans lequel l'échantillon est transporté dans un canal à activité capillaire (2) depuis un endroit d'échantillonnage (3) jusqu'à un endroit de détermination (4), et dans lequel le canal à activité capillaire (2) est formé essentiellement par un support (5), par un recouvrement (6) et par une couche intermédiaire (7) disposée entre le support (5) et le recouvrement (6), qui détermine la géométrie du canal à activité capillaire, le support (5) faisant saillie au-delà du recouvrement (6) dans la zone de l'endroit d'échantillonnage (3), **caractérisé en ce que**
la couche intermédiaire (7) est décalée vers l'arrière dans la zone de l'endroit d'échantillonnage (3) dans la direction de l'endroit de détermination (4), si bien que, aussi bien le support (5) que le recouvrement (6) font saillie au-delà de la couche intermédiaire (7).

2. Dispositif selon la revendication 1, **caractérisé en ce que** le support et la couche intermédiaire ou bien le recouvrement et la couche intermédiaire ou encore le support et le recouvrement et la couche intermédiaire sont réalisés en une seule pièce.

3. Dispositif selon la revendication 1 ou 2, **caractérisé en ce que** le canal à activité capillaire est élargi dans la zone de l'endroit d'échantillonnage.

4. Dispositif selon la revendication 3, **caractérisé en ce que** le canal à activité capillaire est élargi, dans la zone de l'endroit d'échantillonnage, jusqu'à au moins une arête latérale du dispositif.

5. Dispositif selon la revendication 4, **caractérisé en ce que** le canal à activité capillaire est élargi dans la zone de l'endroit d'échantillonnage jusqu'aux deux arêtes latérales du dispositif.

6. Dispositif selon la revendication 3, 4 ou 5, **caractérisé en ce que** le canal à activité capillaire est élargi pour prendre essentiellement la forme d'un entonnoir.

7. Dispositif selon l'une quelconque des revendications précédentes, **caractérisé en ce que**, sur la partie du support faisant saillie au-delà du recouvrement, dans la zone de l'endroit d'échantillonnage, est appliqué un moyen (8) pour la réception de l'échantillon en excès, qui n'entre pas en contact direct avec le recouvrement.

8. Dispositif selon la revendication 7, **caractérisé en ce que** le moyen (8) pour la réception de l'échantillon en excès possède une capillarité inférieure à celle du canal à activité capillaire (2).

9. Dispositif selon la revendication 7 ou 8, **caractérisé en ce que** le moyen (8) contient une fente à activité capillaire ou une matière absorbante.
